(19) Europäisches Patentamt / European Patent Office / Office européen des brevets

(11) **EP 2 491 108 B1**

(12) **FASCICULE DE BREVET EUROPEEN**

(45) Date de publication et mention de la délivrance du brevet:
**04.03.2015 Bulletin 2015/10**

(21) Numéro de dépôt: **10768212.2**

(22) Date de dépôt: **04.10.2010**

(51) Int Cl.:
*C12M 1/34* $^{(2006.01)}$

(86) Numéro de dépôt international:
**PCT/EP2010/064733**

(87) Numéro de publication internationale:
**WO 2011/047954 (28.04.2011 Gazette 2011/17)**

(54) **DISPOSITIF DE CONTRÔLE DE L'ÉVOLUTION D'UNE CULTURE CELLULAIRE**

VERFAHREN ZUR ÜBERWACHUNG DER ENTWICKLUNG VON ZELLKULTUREN

DEVICE FOR MONITORING CELL CULTURE DEVELOPMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.10.2009 FR 0957307**

(43) Date de publication de la demande:
**29.08.2012 Bulletin 2012/35**

(73) Titulaire: **Centre National de la Recherche Scientifique
(CNRS)
75016 Paris (FR)**

(72) Inventeur: **DENOUAL, Matthieu
F-14000 Caen (FR)**

(74) Mandataire: **Regimbeau
Espace Performance Bâtiment K
35769 St-Grégoire Cedex (FR)**

(56) Documents cités:
**EP-A2- 0 036 274        WO-A1-2006/071800
DE-U1-202007 005 399**

• **TLILI C ET AL: "FIBROBLAST CELLS: A SENSING BIOELEMENT FOR GLUCOSE DETECTION BY IMPEDANCE SPECTROSCOPY", ANALYTICAL CHEMISTRY, AMERICAN CHEMICAL SOCIETY, US LNKD- DOI:10.1021/AC0340861, vol. 75, no. 14, 15 juillet 2003 (2003-07-15), pages 3340-3344, XP001176018, ISSN: 0003-2700**

**Description**

L'invention concerne un dispositif de contrôle de l'évolution d'une culture cellulaire.

L'invention se place dans le domaine général de la biologie.

L'invention concerne le contrôle (monitoring) de cultures cellulaires, c'est-à-dire le suivi en temps réel du développement d'une culture cellulaire.

Différentes publications scientifiques sont connues sur le contrôle de cultures cellulaires.

Le document [1] décrit le contrôle de cellules par spectroscopie d'impédance.

Le document [3] concerne la détermination de la conductivité thermique dans des liquides à l'aide d'une sonde de mesure.

Le document [4] décrit un capteur thermique pour contrôler l'interface liquide - gaz dans un micro-canal.

Le document [5] concerne un dispositif de contrôle de l'évolution d'une culture cellulaire, comportant une surface de réception de la culture cellulaire, la surface de réception étant en contact avec une impédance électrique agencée pour avoir une valeur temporelle d'impédance influencée par la présence de cellules.

Les solutions conventionnelles de mesure de paramètres thermiques connues par le document [4], et par exemple la méthode 3-oméga selon le document [2] ne sont pas applicables dans le cas de cultures cellulaires à cause des variations de température néfastes pour la culture qu'elles impliquent. En effet, le principe de ces méthodes est d'appliquer une stimulation de chaleur et de mesurer ensuite la variation de température correspondante.

Le contrôle de culture cellulaire consiste en un suivi quantitatif et/ou qualitatif du développement de cultures cellulaires sur lames ou dans des puits de culture. Ces cultures cellulaires peuvent être soumises à des agents biochimiques extérieurs. L'impact de ces agents sur les cultures peut alors être mis en évidence sur le taux de croissance des cultures et/ou sur la morphologie des cellules. Classiquement, les cultures cellulaires soumises à un agent extérieur sont observées régulièrement dans le temps au microscope et éventuellement comparées à des cultures de références non soumises à l'agent. L'évolution de la population cellulaire au cours du temps et/ou l'évolution de la morphologie des cellules sont une indication de l'effet de l'agent extérieur sur les cellules. Ce type de test permet par exemple de discriminer des agents toxiques d'agents non-toxiques sur tel ou tel type cellulaire.

De façon à multiplier les conditions de test, multi-agent, multi-concentration, multi-type cellulaire, multi-tests pour statistique, des plaques à puits sont utilisées pour mettre en oeuvre plusieurs cultures.

Les législations en rigueur, REACH par exemple, visant à tester la toxicité des produits biochimiques ainsi que les besoins propres au développement de molécules ou de techniques biomédicales tendent à multiplier fortement le nombre de tests à réaliser. La miniaturisation des supports de culture par l'augmentation du nombre de puits par plaques permet de réduire les coûts de tests et d'en augmenter les débits. Par contre, les techniques de contrôle restent à l'heure actuelle basées sur la visualisation par microscopie puis le comptage par analyse d'image:

Pour le contrôle de cultures cellulaires, on connaît des solutions non intégrées et des solutions intégrées.

Les solutions non-intégrées concernent des dispositifs extérieurs de monitoring. Dans ce cas, les cultures sont faites sur plaques avec ou sans puits puis analysées grâce au dispositif externe. La solution la plus répandue et largement majoritaire est l'observation de la culture par microscopie avec ou sans fluorescence. D'autres techniques à l'état de recherche, s'appuient sur des systèmes de détection extérieurs destinés à être immergés dans la solution de culture cellulaire pour évaluer son évolution par le biais de mesure d'impédance, la détection de particules biochimiques, des mesures de champs électriques ou magnétiques.

Les solutions intégrées sont en règle générale à l'état de recherche basées sur l'intégration de solutions non intégrées. On trouve ainsi des dispositifs optiques avec acquisition d'image intégrée sous les puits de culture ou des dispositifs électriques comme les systèmes d'électrodes pour l'impédancemétrie, la détection de particules biochimiques ou les mesures de champs électriques ou magnétiques.

Concernant la solution avec système optique intégré sous les puits de culture, le principal inconvénient est le coût puisque cela revient à intégrer des capteurs optiques de type CCD par exemple sous chacun des puits dans un dispositif à vocation d'utilisation unique (jetable pour des raisons de stérilité). En outre, d'un points de vue réalisation, l'intégration d'un capteur optique n'est pas aisée de façon massive à l'heure actuelle en raison de problèmes de compatibilité entre les procédés plastiques (injection plastique), chauds et sous-pression, et les restrictions quant aux tolérances des systèmes électroniques. De plus, la focalisation pour la netteté de l'image dans ce cas est impossible et donc ce type de dispositif demande une maîtrise très grande du placement du capteur et de la qualité du moulage plastique.

La solution intégrée la plus avancée repose sur le principe électrique d'impédancemétrie selon les documents [1] et [5], c'est-à-dire la mesure de variation d'impédance entre des électrodes pour établir la variation de population cellulaire. La sensibilité du système adapté à la détection de cellule unique dans des micro-canaux est jusqu'à présent limitée dans le cadre d'application de monitoring visant à évaluer l'évolution de cultures cellulaires. Par ailleurs, l'intégration d'électrodes conductrices, souvent opaques, dans les puits limite la transparence du support utile pour l'observation des cellules au microscope pour confirmation visuelle ou observation fine de la morphologie des cellules. D'autre part, l'utilisation de courant dans la solution peut induire des phénomènes perturbant l'évolution de la culture liés aux phénomènes électrochimiques d'oxydation et de réduction ayant lieu au niveau des électrodes libérant des particules. L'évolution même de la culture cellulaire relâchant dans la solution des composants ioniques modifie les conditions de polarisation et interfère avec la mesure. Des solutions utilisant trois électrodes et des matériaux spéciaux sous forme de membrane sont proposées pour pallier ce problème.

L'utilisation de la mesure de champs magnétiques pour suivre une culture cellulaire nécessite l'accrochage de micro/nanoparticules magnétiques sur les cellules de façon les distinguer du milieu de culture. Cet accrochage de particules qui peut s'avérer cher à mettre en oeuvre présent l'inconvénient par ailleurs de perturber les conditions naturelles de croissance. De plus, le signal mesuré, si des particules ne sont pas ajoutées au rythme de la croissance, va diminuer au rythme des divisions cellulaires.

L'invention vise à obtenir un dispositif de contrôle de l'évolution d'une culture cellulaire, permettant d'améliorer les dispositifs connus par l'état de la technique.

A cet effet, un premier objet de l'invention est un dispositif de contrôle de l'évolution d'au moins une culture cellulaire, comportant une surface de réception de la culture cellulaire, la surface de réception étant en contact avec une impédance électrique agencée pour avoir une valeur temporelle d'impédance influencée par la présence de cellules dans la culture cellulaire,

caractérisé en ce que

l'impédance comprend au moins une résistance électrique de chauffage en contact avec la surface de réception pour le chauffage de la culture cellulaire, la résistance étant reliée à un circuit électrique de fourniture d'énergie de chauffage à la résistance, la résistance étant variable en fonction de la température et se trouvant dans une boucle de chaleur formant le circuit électrique de chauffage pour maintenir à une température prescrite de consigne la culture cellulaire, la boucle d'asservissement de chaleur comportant au moins un élément de mesure d'au moins un premier paramètre représentatif de la quantité d'énergie de chauffage consommée par le circuit électrique, cet élément de mesure servant également d'élément de mesure de l'évolution de la culture cellulaire, l'élément de mesure étant distinct de la résistance, le dispositif comportant en outre au moins un calculateur pour calculer à partir du premier paramètre représentatif de la quantité d'énergie de chauffage consommée par le circuit électrique au moins un deuxième paramètre représentatif de l'évolution temporelle de la culture cellulaire.

Suivant d'autres caractéristiques de l'invention,

- Le dispositif comporte un générateur d'un signal prédéterminé de stimulation thermique extérieure relié à un point déterminé de la boucle d'asservissement de chaleur, le calculateur étant prévu pour calculer à partir du premier paramètre représentatif de la quantité d'énergie de chauffage consommée par le circuit électrique au moins un troisième paramètre thermique, le troisième paramètre thermique étant calculé par le calculateur à partir de la réponse de la résistance au signal de stimulation thermique extérieure, ledit deuxième paramètre représentatif de l'évolution temporelle de la culture cellulaire étant calculé par le calculateur à partir dudit troisième paramètre thermique.
- Le paramètre thermique comprend la capacité thermique de la résistance lorsqu'elle est en présence de la culture cellulaire et/ou la conductance thermique de la résistance lorsqu'elle est en présence de la culture cellulaire.
- Le signal de stimulation thermique extérieure est appliqué à l'une des bornes de la résistance par l'intermédiaire d'au moins une capacité électrique.
- Le signal de stimulation thermique extérieure est appliqué à un module additionneur comportant une première entrée sur laquelle se trouve le signal de mesure, une deuxième entrée reliée au point déterminé de la boucle d'asservissement de chaleur, auquel est appliqué le signal de stimulation extérieure, et une sortie fournissant un signal d'asservissement de la résistance égal à la somme des signaux présents sur la première entrée et sur la deuxième entrée.
- Le signal de stimulation thermique extérieure est sous la forme d'un signal électrique ayant une composante fréquentielle de moyenne nulle.
- Ou le signal de stimulation thermique extérieure est sous la forme d'un signal électrique impulsionnel.
- La résistance électrique de chauffage forme au moins en partie la surface de réception de la culture cellulaire.
- Ou la surface de réception de la culture cellulaire est prévue sur un matériau électriquement isolant interposé entre la résistance électrique de chauffage et la culture cellulaire.
- Le matériau électriquement isolant interposé entre la résistance électrique de chauffage et la culture cellulaire est fixé de manière amovible par rapport à la résistance électrique de chauffage.
- La résistance est métallique avec une épaisseur comprise entre 5 et 20 nanomètres.
- La résistance comporte une première extrémité reliée à un premier contact électrique et une deuxième extrémité reliée à un deuxième contact électrique, la résistance ayant entre la première extrémité et la deuxième extrémité une valeur résistive variable en fonction de la température, la valeur résistive de la résistance étant supérieure à la valeur résistive des contacts électriques formés chacun par au moins une couche métallique d'une épaisseur totale comprise entre 50 et 150 nanomètres.
- La résistance électrique de chauffage forme au moins en partie le fond ou est disposée sous le fond d'au moins un puits de culture contenant ladite au moins une culture cellulaire.
- Une pluralité de puits de culture destinés à contenir une pluralité de cultures cellulaires est disposée sur un support comportant une pluralité de résistances électriques de chauffage des cultures cellulaires, les résistances électriques de chauffage étant agencées pour former au moins en partie le fond des puits de culture ou étant disposées sous le fond des puits de culture.
- La température prescrite de consigne est constante et égale à une température déterminée de croissance de cellules vivantes dans la culture cellulaire.
- Le deuxième paramètre représentatif de l'évolution temporelle de la culture cellulaire est l'aire couverte par les cellules de la culture cellulaire sur la surface de réception ou le pourcentage d'aire couverte par les cellules de la culture cellulaire par rapport à l'aire de la surface de réception.

Un deuxième objet de l'invention est un procédé de contrôle de l'évolution d'au moins une culture cellulaire à l'aide du dispositif de contrôle tel que décrit ci-dessus, dans lequel une culture cellulaire est disposée sur une surface de réception de la culture cellulaire, la surface de réception étant en contact avec une impédance électrique agencée pour avoir une valeur temporelle d'impédance influencée par la présence de cellules dans la culture cellulaire, caractérisé en ce que l'impédance comprend une résistance électrique de chauffage, l'on chauffe par la résistance électrique de chauffage en contact avec la surface de réception la culture cellulaire, la résistance étant reliée à un circuit électrique de fourniture d'énergie de chauffage à la résistance, la résistance étant variable en fonction de la température et se trouvant dans une boucle d'asservissement de chaleur formant le circuit électrique de chauffage pour maintenir à une température prescrite de consigne la culture cellulaire, on mesure par un élément de mesure de la boucle d'asservissement de chaleur au moins un premier paramètre représentatif de la quantité d'énergie de chauffage consommée par le circuit électrique, cet élément de mesure servant également d'élément de mesure de l'évolution de la culture cellulaire, l'élément de mesure étant distinct de la résistance, on calcule par au moins un calculateur à partir du premier paramètre représentatif de la quantité d'énergie de chauffage consommée par le circuit électrique au moins un deuxième paramètre représentatif de l'évolution temporelle de la culture cellulaire.

L'invention sera mieux comprise à la lecture de la description qui va suivre, donnée uniquement à titre d'exemple non limitatif en référence aux dessins annexés, sur lesquels :

- la figure 1 est une vue schématique en coupe verticale d'une surface réception de culture cellulaire, associée à une résistance de chauffage, selon un mode de réalisation,
- la figure 2 est un schéma électrique du dispositif de contrôle suivant l'invention,
- la figure 3 est un schéma électrique du dispositif de contrôle suivant l'invention, selon un premier mode de réalisation,
- la figure 4 est un schéma électrique du dispositif de contrôle selon l'invention, selon un deuxième mode de réalisation,
- la figure 5 est un schéma électrique du dispositif de contrôle selon l'invention, selon un troisième mode de réalisation,
- la figure 6 est un schéma électrique du dispositif de contrôle selon l'invention, selon une variante du troisième mode de réalisation,
- la figure 7 est un schéma équivalent de la résistance du dispositif en boucle ouverte,
- la figure 8 est un schéma équivalent de la résistance du dispositif en boucle fermée.

Aux figures, un puits 2 de réception d'une culture cellulaire CC est prévu au-dessus d'une résistance électrique 1 de chauffage de la culture CC. Le puits 2 est délimité latéralement par des parois 20, par exemple verticales. La résistance 1 se trouve sur un support isolant 100, par exemple en étant photogravée sur la surface supérieure 101 du support isolant 100 pour former un circuit imprimé. La résistance 1 comporte une première extrémité 110 reliée à un premier contact électrique 4 et une deuxième extrémité 120 reliée à un deuxième contact électrique 5. Entre la première extrémité 110 et la deuxième extrémité 120, la résistance électrique 1 possède une valeur résistive R en ohms variable en fonction de la température. La résistance électrique 1 se trouve au moins en partie sous la culture cellulaire CC.

La culture cellulaire CC contient des cellules vivantes C cultivées dans un milieu liquide nutritif, prévu pour la croissance des cellules C. D'une manière usuelle, ce milieu de culture est constitué à environ 90% d'eau, de nutriments et d'oligo-éléments devant être consommés pour faire croître et se multiplier les cellules. La plupart des cellules C se déposent par sédimentation au fond du puits 2. Bien entendu, il peut avoir été ajouté à la culture cellulaire CC toute substance dont on souhaite détecter l'impact sur les cellules.

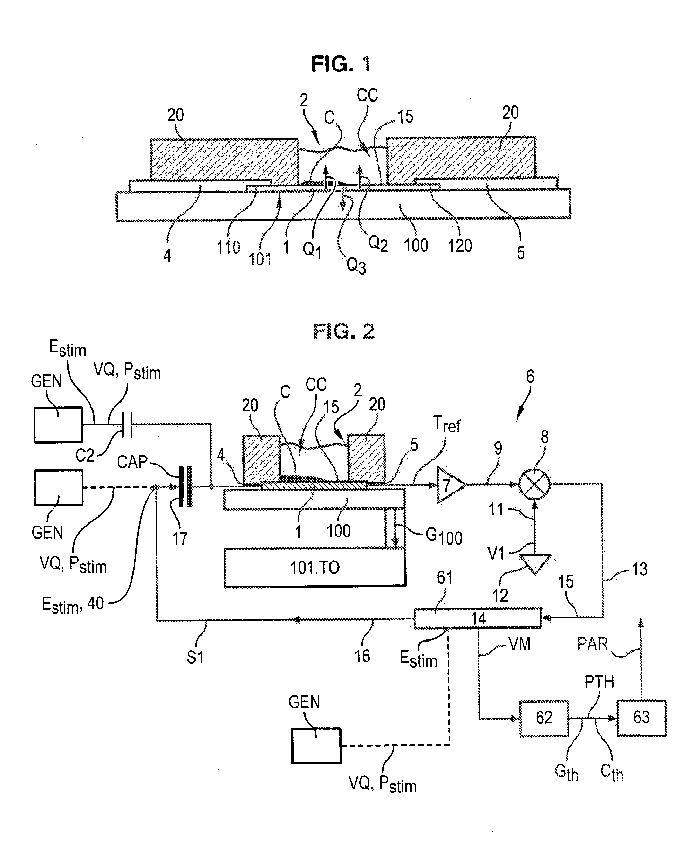

Le dispositif forme un capteur des cellules C dans la culture CC. Dans le mode de réalisation représenté à la figure 1, la surface supérieure 15 de la résistance 1 forme au moins en partie la surface de réception de la culture cellulaire C, c'est-à-dire est en contact direct avec cette culture cellulaire CC liquide.

Dans le mode de réalisation représenté à la figure 1, tout le fond du puits 2 est formé par la surface supérieure 15 de la résistance 1.

Un puits 2 de culture habituel fait par exemple une aire de 1 cm$^2$ de fond sur la résistance 1 pour une hauteur d'environ 1 cm, et représente un volume d'environ 1 cm$^3$.

Les contacts électriques 4 et 5 forment chacun une plage conductrice ayant une valeur résistive inférieure à la valeur résistive R de la résistance 1. La résistance 1 est par exemple en métal, pouvant être de l'or ou du platine, en une épaisseur totale comprise entre 5 et 20 nanomètres, par exemple pour chauffer une surface de culture de 1 cm$^2$. Les contacts électriques 4 et 5 sont par exemple formés par une ou plusieurs couches métalliques d'une épaisseur totale comprise entre 50 et 150 nanomètres. Par exemple, les contacts électriques 4 et 5 ont chacun une partie respectivement 41, 51 recouvrant les extrémités 110 et 120 de la résistance 1.

La résistance 1 est reliée par ses contacts électriques 4 et 5 à une boucle 6 d'asservissement de chaleur formant un circuit électrique de chauffage de la résistance 1. Cette boucle 6 d'asservissement de chaleur est par exemple du type de celle décrite dans le document WO 2009/034066 A1. La boucle 6 d'asservissement de chaleur est prévue pour maintenir la température de la résistance 1 égale à une température Tref de consigne et pour chauffer la résistance 1.

La température prescrite Tref de consigne est la température nécessaire à la vie des cellules C dans la culture CC et est par exemple constante. Cette température Tref de consigne est par exemple de 37°C. Cette température Tref est celle prévue pour permettre la croissance normale des cellules C dans la culture CC en présence du milieu de culture contenu dans le puits 2. La boucle 6 maintient donc la température de la résistance 1, de la surface 15 et la culture CC à une température constante. La boucle 6 comporte un correcteur 14 d'application d'une puissance de chauffage à la résistance 1 pour maintenir la température de la résistance 1 à la température Tref de consigne. Par exemple, ainsi que cela sera décrit ci-après, le correcteur 14 est prévu pour générer une composante fréquentielle S1 de la puissance de chauffage, qui est appliquée à un premier moyen 17 de couplage prévu entre la résistance 1 et le correcteur 14 pour appliquer à la résistance 1 un signal sans composante continue, un deuxième moyen 19 de couplage, distinct du premier moyen 19 de couplage, est prévu entre la résistance 1 et un moyen 18 de polarisation continue pour maintenir la résistance électrique 1 à un point de fonctionnement continu prescrit.

Bien entendu, la température prescrite Tref de consigne pourrait être différente de la température de vie ou de croissance des cellules C dans la culture CC, par exemple à des fins de test des cellules C à cette température différente.

La boucle 6 d'asservissement de chaleur comporte un élément 61 de mesure de la quantité d'énergie de chauffage consommée pour maintenir la résistance 1 à la température prescrite Tref de consigne. Cet élément 61 de mesure est donc distinct de la résistance 1 servant au chauffage de la culture cellulaire CC. L'élément 61 fournit un signal VM de mesure de la quantité d'énergie de chauffage consommée. Ce signal VM est un premier paramètre représentatif de la quantité d'énergie de chauffage consommée par le circuit électrique pour maintenir la température Tref prescrite de consigne.

Un calculateur 62 calcule à partir du signal VM de mesure de la quantité d'énergie de chauffage consommée, fourni par l'élément 61 de mesure, un deuxième paramètre PAR représentatif de l'évolution temporelle de la culture cellulaire CC.

On extrait les paramètres thermiques PTH de la surface 15 de culture cellulaire sans en modifier la température. Pour ce faire, le calculateur 62 calcule d'abord les paramètres thermiques de la résistance 1 chauffant la culture cellulaire CC, ces paramètres thermiques comportant par exemple la capacité thermique $C_{th}$ et/ou la conductance thermique $G_{th}$ vue par la résistance 1 au cours du temps lorsqu'elle est soumise à la culture cellulaire CC.

En effet, la quantité de chaleur que devra fournir la résistance 1 de chauffage à la culture cellulaire CC ne sera pas la même selon qu'il y a plus ou moins de cellules C dans le puits 2 et notamment au fond du puits 2.

En effet, l'inventeur s'est aperçu que plus il y avait de cellules C vivantes au fond du puits 2 et moins il fallait chauffer la culture CC et moins il fallait que la résistance 1 apporte de chaleur.

On utilise ainsi la mesure de la variation des propriétés thermiques de la surface 15 lors de l'évolution de la population cellulaire C. Lors de leur croissance, les cellules C couvrent la surface 15 ou le fond du puits 2 et les transferts de chaleur entre la surface 15 et le milieu CC de culture sont alors différents suivant la présence ou non de cellules entre ces derniers. Ainsi, la mesure des paramètres thermiques, conductance et capacité, au niveau de la surface 15 donne une indication de l'évolution de la population cellulaire C.

Ainsi, en l'absence de cellules C, l'interface entre la surface 15 et le milieu CC de culture est le siège d'un phénomène de convexion de chaleur $Q_2$ allant de la résistance 1 (surface de chauffage) au fluide CC (milieu de culture) ainsi que cela est représenté à la figure 1. En présence de cellules C, l'échange se fait par conduction thermique entre la surface 15 de la résistance 1 et les cellules C, puis par convection entre les cellules C et le milieu CC de culture, ainsi que cela est représenté globalement par le flux de chaleur $Q_1$ à la figure 1. Ces phénomènes thermiques de natures différentes impliquent des caractéristiques thermiques différentes pour l'environnement de la surface de culture suivant la présence ou l'absence de cellules. Ainsi, en déterminant l'évolution des paramètres thermiques de la surface de culture au cours du temps, on suit l'évolution de la population cellulaire C au niveau de la surface de culture. A la figure 1, le flux de chaleur $Q_3$ représente la chaleur fournie par la résistance 1 de chauffage au support 100 ayant une conductivité thermique $G_{100}$ déterminée. Dans ce qui suit la surface 15 de la résistance 1 tournée vers la culture cellulaire CC est appelée surface 15 de culture cellulaire.

On décrit ci-dessous un exemple de calcul par le calculateur 62 d'un troisième paramètre thermique Pth que sont la capacité thermique $C_{th}$ et/ou la conductance thermique $G_{th}$.

L'analyse de la réponse du système global (boucle fermée) à une stimulation extérieure de puissance $P_{stim}$ sur une entrée $E_{stim}$ du circuit électrique va permettre d'accéder aux grandeurs recherchées qui sont la conductance et la capacité thermique, comme nous le détaillons ci-dessous. La détection des cellules C par le dispositif est thermique. Cette stimulation extérieure $P_{stim}$ est apportée par un générateur extérieur GEN relié électriquement à boucle 6. L'entrée $E_{stim}$ est donc reliée au générateur GEN.

Considérons le système thermique ouvert constitué de la surface 15 de culture cellulaire. Les paramètres thermiques, conductance $G_{th}$ et capacité $C_{th}$, de la surface 15 de culture cellulaire, évoluent dans le temps en fonction de la croissance des cellules.

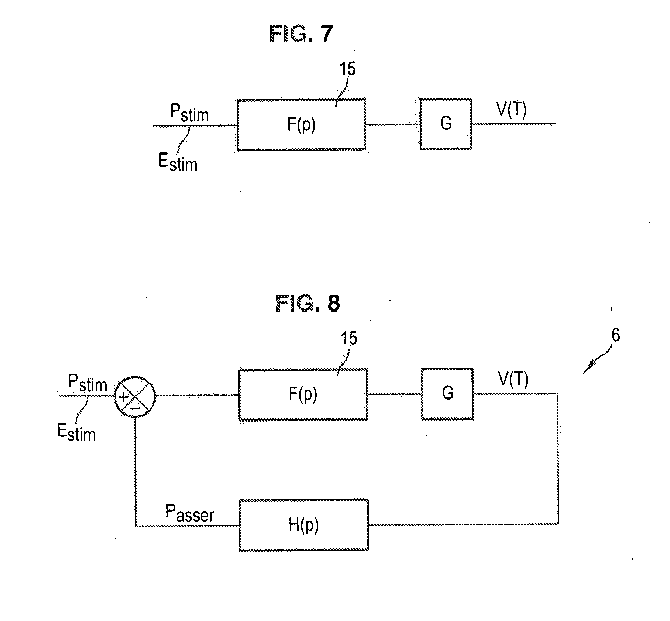

Soit F(p) la représentation du système thermique à la figure 7 :

$$F(p) = \frac{A}{G_{th}(1 + \tau_{th}\, p)}$$

Avec

$$\tau_{th} = \frac{C_{th}}{G_{th}}$$

G représente le gain de l'électronique de conditionnement et le transfert de mesure de la température.

Avec un correcteur classique H(p) de type proportionnel-intégral dans la boucle 6 à la figure 8 :

$$H(p) = \frac{1 + Kp}{p}$$

le système bouclé global, c'est-à-dire la boucle 6, devient :

$$P_{asser}(p) = \frac{A(1 + Kp)}{1 + \dfrac{G + AK}{A}\, p + \dfrac{C_{th}}{A}\, p^2}\, P_{stim}(p)$$

avec F(p) recevant en entrée la différence ($P_{stim}$ (p) - $P_{asser}$(p)) et H(p) en série avec F(p) et G.

L'analyse des régimes transitoires (dépassements, oscillation, amortissement) en cas de stimulation de type impulsionnel ou de la réponse fréquentielle en cas de stimulation de type fréquentiel permettra de remonter aux para-mètres $C_{th}$ et $G_{th}$ et ainsi à la couverture de la surface 15 de culture par les cellules.

Un système correcteur adaptatif peut être envisagé pour maintenir constantes les performances d'asservissement.

Etant donné la lenteur des phénomènes à suivre - croissance de cellules -, de multiples acquisitions pourront être faites et moyennées pour améliorer la résolution.

Du fait de son concept simple, cette méthode de suivi de croissance cellulaire peut facilement être parallélisée pour être combinée à des dispositifs de type plaque à plusieurs puits.

La stimulation extérieure $P_{stim}$ est par exemple une variation de chaleur VQ. On applique le signal de stimulation

extérieure $P_{stim}$ sous la forme d'un signal électrique $P_{stim}$ à un point déterminé de la boucle 6.

Comme stimulation extérieure $P_{stim}$, la variation de chaleur VQ est appliquée sur la borne 4 par exemple sous la forme d'un signal électrique $P_{stim}$ par l'intermédiaire d'une capacité électrique C2, dont la borne autre que celle reliée à la borne 4 forme l'entrée $E_{stim}$. On peut également envisager d'utiliser le moyen 17 de couplage ou capacité 17 décrite ci-dessous en additionnant électriquement le signal de stimulation extérieure $P_{stim}$ au signal d'asservissement S1, par exemple en appliquant le signal de stimulation extérieure $P_{stim}$ directement à la capacité 17 en sa borne 40 autre que celle reliée à la borne 4, cette borne 40 de la capacité 17 formant l'entrée $E_{stim}$.

Dans un mode de réalisation préféré, le signal de stimulation extérieure $P_{stim}$ est additionné au signal VM pour fournir la somme résultante à l'entrée 144 qui sera décrite ci-dessous et qui sert à obtenir le signal S1 d'asservissement de la résistance 1 dans la boucle 6. Le correcteur 14 comporte donc dans ce cas l'entrée $E_{stim}$, à laquelle est appliqué le signal de stimulation extérieure $P_{stim}$, et un module 200 d'addition de cette entrée $E_{stim}$, qui sera décrit ci-dessous.

Le signal $P_{stim}$ appliqué sur l'entrée $E_{stim}$ est par exemple du même genre que le signal S1 qui sera décrit ci-dessous. Le signal $P_{stim}$ appliqué sur l'entrée $E_{stim}$ est par exemple un signal électrique alternatif ayant une composante fréquentielle à moyenne nulle, pouvant être haute fréquence, par exemple dans une bande de fréquence dont la limite inférieure est d'au moins 20 kHz. Le signal électrique $P_{stim}$ appliqué sur l'entrée $E_{stim}$ peut être sinusoïdal. Le signal électrique $P_{stim}$ appliqué sur l'entrée $E_{stim}$ peut également être impulsionnel. La stimulation extérieure $P_{stim}$, VQ sur l'entrée $E_{stim}$ sert au chauffage de la résistance 1. Dans ce qui suit ce signal électrique $P_{stim}$, VQ s'ajoute au signal S1 aux bornes de la résistance 1. On applique donc à la boucle 6 le signal VQ, $P_{stim}$ prédéterminé de stimulation thermique extérieure.

A partir du ou des paramètres thermiques Pth, tels que par exemple la capacité thermique $C_{th}$ et la conductance thermique $G_{th}$, un calculateur 63 calcule le deuxième paramètre PAR représentatif de l'évolution temporelle de la culture cellulaire, ce paramètre PAR étant par exemple l'aire couverte par les cellules C dans le fond du puits ou le pourcentage de couverture des cellules par rapport à l'aire totale du fond du puits 2.

Par exemple, les signaux utilisés par les calculateurs, par exemple VM, sont convertis d'analogique en numérique par un convertisseur analogique-numérique. Les calculateurs 62 et 63 peuvent ne faire qu'un et être mis en oeuvre par exemple par un ordinateur. Les calculateurs comportent des moyens automatiques de calcul à cet effet.

On décrit ci-dessous différents modes de réalisation de la boucle 6. On utilise le principe de substitution électrique par couplage capacitif.

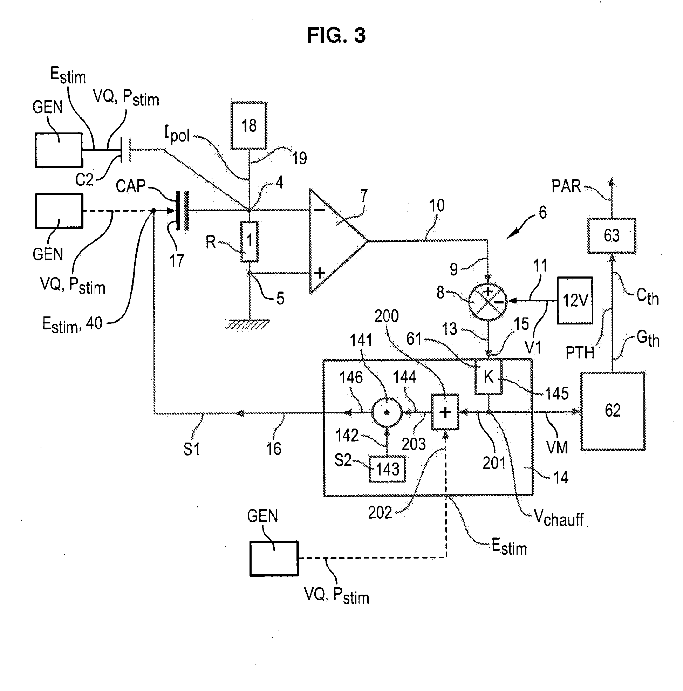

Dans un premier mode réalisation représenté aux figures 2 et 3, la boucle 6 comporte : un dispositif 7 d'amplification de la tension aux bornes 4, 5 de la résistance 1, un élément soustracteur 8 comportant une première entrée 9 reliée à la sortie 10 de tension du dispositif 7 d'amplification et une deuxième entrée 11 reliée à un module 12 imposant une tension VI de consigne constante prescrite, pour former sur une sortie 13 du soustracteur 8 la différence Vchauff ou signal d'erreur entre les deux entrées 9, 11. La sortie 13 du soustracteur 8 est reliée à l'entrée 15 d'un module correcteur 14 appliquant par sa sortie 16 un signal alternatif S1 qui est fonction du signal présent sur son entrée 15. La sortie 16 est reliée par un premier moyen 17 de couplage à la résistance 1.

A la figure 3, un module 18, distinct de la boucle 6 d'asservissement de chaleur, est prévu pour imposer une polarisation prescrite Ipol du point de fonctionnement de la résistance 1 en courant continu par l'intermédiaire d'un deuxième moyen 19 de couplage, tandis que le premier moyen 17 de couplage applique à la résistance 1 une composante fréquentielle S1 à moyenne nulle. Par exemple, à la figure 3, les moyens 17, 19 de couplage sont reliés à un même noeud formé par la première borne 4 de la résistance 1, dont la deuxième borne 5 est reliée à la masse.

Le signal S1 fréquentiel sert au chauffage de la résistance 1.

La contre-réaction de chaleur par le signal S1 de chauffage asservit la température de la résistance 1 à la température Tref, sans en modifier le point de fonctionnement lié au courant Ipol de polarisation la traversant.

Dans un mode de réalisation, le ou les éléments résistifs de chauffage de la culture CC dans la boucle 6 ne comprennent que la résistance 1.

Dans le mode de réalisation représenté aux figures, le premier moyen 17 de couplage est capacitif et comprend un ou plusieurs condensateurs formant une capacité CAP de valeur déterminée. Le deuxième moyen 19 de couplage est formé par de simples conducteurs électriques.

Le signal S1 de chauffage est par exemple un signal haute fréquence, dans une bande de fréquence supérieure à 20 kHz.

Dans un mode de réalisation, le correcteur 14 forme un premier signal S1 de chauffage, qui est sinusoïdal et par exemple proportionnel au signal sur l'entrée 15. Le signal S1 de chauffage est par exemple formé par modulation d'amplitude du signal de différence Vchauff par un deuxième signal sinusoïdal S2 ayant ladite fréquence. Le correcteur 14 comprend par exemple à cet effet un multiplicateur 141 comportant une première entrée 142 reliée à un module 143 de fourniture du deuxième signal sinusoïdal $\sin(\omega.t)$, une deuxième entrée 144 reliée à l'entrée 15, par exemple par l'intermédiaire d'un module 145 apportant un facteur multiplicatif K constant prescrit et éventuellement par l'intermédiaire du module additionneur optionnel 200 qui sera décrit ci-dessous, et une sortie 146 reliée à la sortie 16. Les éléments 141, 143 forment un moyen de modulation du signal intermédiaire sur l'entrée 144 par le signal sinusoïdal S2 pour former le signal alternatif S1.

On suppose ci-dessous que l'entrée 144 est reliée directement au module 145 sans module additionneur 200. On a par exemple dans ce cas :

$$S1 = Vchauff. \sin(\omega.t)$$

avec

Vchauff le signal présent sur l'entrée 144,
t le temps,
$\omega$ une pulsation prescrite.

Le courant traversant la résistance R de mesure est alors :

i(t) = Ipol + (Vchauff / R).sin(co.t)

La valeur moyenne de i(t) est égale à Ipol.
La puissance p(t) dans la résistance 1 est alors :

$$p(t) = R.\{ Ipol2 + 2.Ipol.(Vchauff / R).\sin(\omega.t) + ((Vchauff / R).\sin(\omega.t))2\}$$

et a une valeur moyenne

$$\overline{p(t)} = R.I_{pol}{}^2 + \frac{1}{2}\frac{V_{chauff}{}^2}{R}$$

On peut donc faire évoluer la puissance dissipée dans la résistance 1, et par conséquent sa température, sans changer son point de fonctionnement, étant donné que le courant moyen traversant la résistance de mesure reste constant à Ipol.

A la figure 2, l'élément 61 de mesure est par exemple formé par le correcteur 14. Plus précisément, à la figure 3, cet élément 61 de mesure de la quantité d'énergie de chauffage est formé par l'élément 145 fournissant le signal $V_{chauff}$. Ce signal $V_{chauff}$ est représentatif de la quantité d'énergie fournie par la boucle 6 à la résistance 1. Ce signal $V_{chauff}$ est envoyé du correcteur 14 à un calculateur 62, qui calcule à partir de ce signal de quantité d'énergie le paramètre PAR représentatif de l'évolution temporelle de la culture cellulaire CC.

Dans le cas où le module additionneur 200 est prévu à la figure 3, ce module additionneur 200 comporte une première entrée 201 reliée au signal $V_{chauff}$ et au module 145, une deuxième entrée 202 reliée à l'entrée $E_{stim}$ de signal $P_{stim}$ de stimulation extérieure, et une sortie 203 reliée à l'entrée 144. Le module additionneur 200 fournit sur sa sortie 203 et donc sur l'entrée 144 la somme du signal $V_{chauff}$ et du signal $P_{stim}$ de stimulation extérieure.

Bien entendu, plusieurs résistances 1 de chauffage peuvent être prévues pour chauffer plusieurs puits 2 de culture, les résistances 1 étant connectées dans le même circuit électrique ou dans des circuits électriques distincts. Par exemple, plusieurs puits 2 avec chacun leur boucle 6 associée peuvent être prévus sur le même support 100. Plusieurs résistances 1 peuvent être prévues en parallèle dans le même circuit électrique.

Dans d'autres modes de réalisation, la ou les résistances 1 de chauffage pourront être isolées électriquement du milieu de culture CC. Etant donné les ordres de grandeur des résistances nécessaires, de l'ordre de la centaine d'Ohm environ, l'épaisseur des couches métalliques employées pour les réaliser sera plus faible (5 à 20 nanomètres) que celle utilisée classiquement pour faire des électrodes (>150 nanomètres). Cette couche métallique est par exemple en or ou en platine. Cela rend moins coûteux le procédé de fabrication, d'une part car moins de métaux sont utilisés, d'autre part car cette faible épaisseur permet même de garantir une semi-transparence du support rendant ainsi possible l'observation par microscope à transmission lors des cultures. Des configurations d'éléments résistifs 1 de surface sous forme de grille peuvent également être envisagées.

Dans d'autres modes de réalisation non représentés, une couche très fine d'un matériau est prévue entre la surface supérieure 15 de la résistance 1 et la culture cellulaire CC, cette couche formant donc le fond du puits 2 et donc la surface de réception de la culture cellulaire. Cette couche est par exemple prévue pour empêcher un contact direct de la culture cellulaire CC avec la résistance 1, dans le cas où le matériau de la résistance est susceptible d'entrer en interaction avec les cellules ou la culture cellulaire.

Dans un mode de réalisation, le dispositif se présente sous la forme d'un boîtier électronique ayant une interface informatique associée à des lames de culture jetables couvertes de plusieurs puits de culture ou non et présentant une ou des surfaces isolées électriquement des cellules pour la mise en oeuvre de l'asservissement de chaleur par substitution électrique et couplage capacitif pour l'extraction des paramètres électriques. Le boîtier implémente l'asservissement de chaleur par substitution électrique et couplage capacitif, tandis que les lames de culture jetables constitueront un dispositif purement passif à faible coût facilement industrialisable.

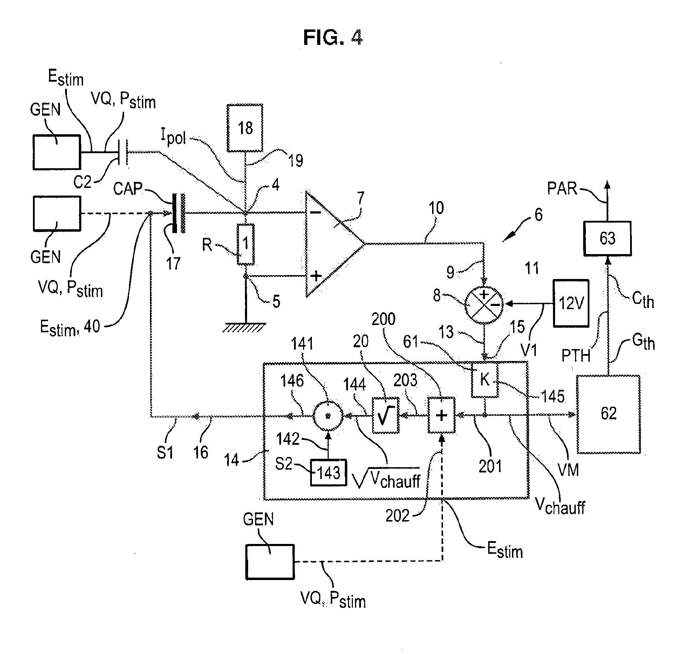

Dans un deuxième mode de réalisation représenté à la figure 4 et conforme au premier mode de réalisation des figures 2 et 3, il y a en plus un moyen 20 permettant de linéariser la puissance du signal S1 en fonction du signal de différence $V_{chauff}$ ou du signal de température mesuré par l'élément 1. A la figure 4, ce moyen 20 de linéarisation a par exemple la forme d'un module de formation de racine carrée disposé en amont du multiplicateur 141 de modulation d'amplitude et en aval de l'entrée 15, par exemple entre le module 145 et le multiplicateur 141 (ou en variante entre le module 145 et l'entrée 15). Le module 20 forme ainsi sur l'entrée 144 un signal proportionnel à

$$\sqrt{V_{chauff}}$$

dans le cas où l'entrée 144 est reliée directement au module 145. La puissance P(S1) du signal S1 appliqué par le moyen 17 à l'élément 2 est alors proportionnelle au signal de différence $V_{chauff}$. A la figure 3, l'élément 61 de mesure de la quantité d'énergie de chauffage est formé par l'élément 145 fournissant le signal $V_{chauff}$, qui est représentatif de la quantité d'énergie fournie par la boucle 6 à la résistance 1. Dans le cas où le module additionneur 200 est prévu, ce module 200 tel qu'il est décrit ci-dessus est par exemple prévu en amont du module 20 ainsi que représenté, pour fournir sur l'entrée 144 un signal proportionnel à la somme $\sqrt{\left(V_{chauff} + P_{stim}\right)}$, l'entrée du module 20 étant reliée à la sortie 203 de ce module additionneur 200 et la deuxième entrée 202 de ce module additionneur 200 étant reliée à l'entrée $E_{stim}$ de signal $P_{stim}$ de stimulation extérieure. Bien entendu, le module additionneur 200 pourrait également être prévu en aval du module 20, avec la sortie 203 reliée directement à l'entrée 144 et la première entrée 201 reliée à la sortie du module 20.

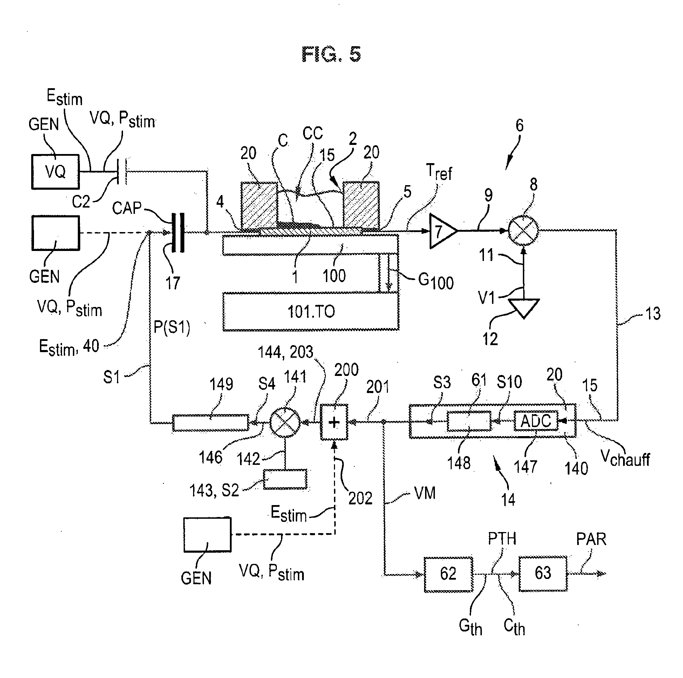

Dans le troisième mode de réalisation représenté à la figure 5, le moyen 20 de linéarisation de la puissance du signal S1 en fonction du signal de différence $V_{chauff}$ possède une autre forme. Le signal de différence $V_{chauff}$ est numérisé dans le correcteur numérique 14. Le signal de différence peut également être formé dans le contrôleur par numérisation du signal 9 et calcul de la différence avec une valeur mémorisée dans le contrôleur. Ce correcteur 14 est mis en oeuvre par un microcontrôleur 140, un microprocesseur, un microordinateur, ou autres. A cet effet, le signal de différence $V_{chauff}$ est envoyé dans le correcteur 14 à un convertisseur 147 analogique - numérique (ADC) fournissant à partir du signal de différence $V_{chauff}$ analogique présent sur l'entrée 15 du correcteur 14 un signal numérique S10. Ce signal numérique S10 est modulé en largeur d'impulsion (PWM pour "pulse with modulation" en anglais) par un module 148 de modulation de largeur d'impulsion, comportant par exemple un correcteur proportionnel et intégral PI pour appliquer une correction proportionnelle et intégrale au signal S10, pour former un troisième signal S3 intermédiaire, dit signal de rétroaction de chauffage. Le rapport cyclique β du troisième signal S3 intermédiaire modulé en largeur d'impulsion est égal, exprimé en %, à

$$\beta = 100.tON/T$$

où

tON est le temps pendant lequel le signal S3 se trouve à un premier niveau, par exemple le niveau haut 1,
T est la période prescrite et constante du signal S3,
tOFF = T - tON est le temps pendant lequel le signal S3 se trouve à un deuxième niveau différent du premier niveau, ce deuxième niveau étant par exemple le niveau bas. La valeur moyenne du troisième signal S3 intermédiaire modulé en largeur d'impulsion est égale à la valeur du signal en sortie du contrôleur.

Le correcteur 14 fournit sur l'entrée 144 le troisième signal S3 intermédiaire. L'entrée 144 est reliée par exemple directement ci-dessous au multiplicateur 141 pour effectuer la modulation d'amplitude du troisième signal intermédiaire S3 par le deuxième signal sinusoïdal S2 de porteuse du module 143, afin de fournir sur la sortie 146 un quatrième signal S4 sinusoïdal pour un seul des premier et deuxième niveau de S3, par exemple pour le premier niveau correspondant à tON et un signal constant, par exemple nul, pour l'autre niveau de S3, par exemple pour le deuxième niveau correspondant à tOFF. Le quatrième signal S4 est ensuite envoyé au moyen 17 de couplage pour former le signal S1 fréquentiel par exemple par l'intermédiaire d'un filtre passe-bande 149 autour de la fréquence de la porteuse S2.

La valeur numérique calculée par le contrôleur proportionnel et intégral en fonction du signal S10 est égale à la valeur moyenne du signal S3, c'est-à-dire égale à β. Vmax.

avec Vmax la tension maximale à la sortie du composant numérique pour un rapport cyclique β de 100%.

$$S2 = Kampl \cdot \sin(\omega.t)$$

S4 = Kmult .S3 .S2 = Kmult. Kampl .sin($\omega$.t) . S3
avec Kampl une constante et Kmult une constante liée au multiplicateur.

La valeur efficace du signal S4 est

$$V_{RMS}(S4) = \sqrt{\frac{1}{T}\int_0^T (K_{mult} \cdot S3 \cdot S2)^2 dt}$$

soit

$$V_{RMS}(S4) = \sqrt{\frac{1}{T}\int_0^{\beta T} (K^2{}_{mult} \cdot V^2{}_{max} \cdot S2)^2 dt}$$

$$V_{RMS}(S4) = \sqrt{\frac{\beta}{2} K^2{}_{mult} \cdot V^2{}_{max} \cdot K^2{}_{ampl}}$$

$$V_{RMS}(S4) \propto \sqrt{\beta}$$

$$P(S1) = \frac{V^2{}_{RMS}(S4)}{R_{mes}} \propto \beta \propto S3 \propto V_{chauff}$$

où P(S1) est la puissance du signal S1 et le signe ∝ signifie la proportionnalité.

La puissance de contre-réaction appliquée sur la résistance 1 est donc directement proportionnelle à la valeur calculée à la sortie du contrôleur 148. Le système est alors entièrement linéaire. On n'a plus besoin de faire d'hypothèse pour la linéarisation et le calcul des correcteurs.

La modulation par largeur d'impulsion permet ainsi une linéarisation de la puissance du signal S1 en fonction du signal de différence Vchauff. La linéarisation pourrait bien sûr être mise en oeuvre comme en analogique par l'utilisation d'une fonction racine carrée numérique, mais l'utilisation d'une modulation par largeur d'impulsion PWM permet directement la linéarisation sans fonction complémentaire ou autre calcul.

Dans le cas où le module additionneur 200 est prévu, ce module 200 tel qu'il est décrit ci-dessus est par exemple prévu en amont du module 141 et en aval du module 148 ainsi que représenté à la figure 5, l'entrée 144 étant reliée à la sortie 203 de ce module additionneur 200, l'entrée 201 étant reliée à la sortie du module 148 fournissant le signal S3 et l'entrée 202 étant reliée à l'entrée $E_{stim}$ de signal $P_{stim}$ de stimulation extérieure. Le module additionneur 200 fournit sur sa sortie 203 et donc sur l'entrée 144 la somme du signal S3 et du signal $P_{stim}$ de stimulation extérieure.

A la figure 5, l'élément 61 de mesure de la quantité d'énergie de chauffage est formé par l'élément 148 fournissant sur sa sortie le signal S3 qui est représentatif de la quantité d'énergie fournie par la boucle 6 à la résistance 1.

Bien entendu, dans les modes de réalisation décrits ci-dessus, la modulation d'amplitude peut être faite avec un multiplieur 141 ou un simple interrupteur, par exemple à transistor(s).

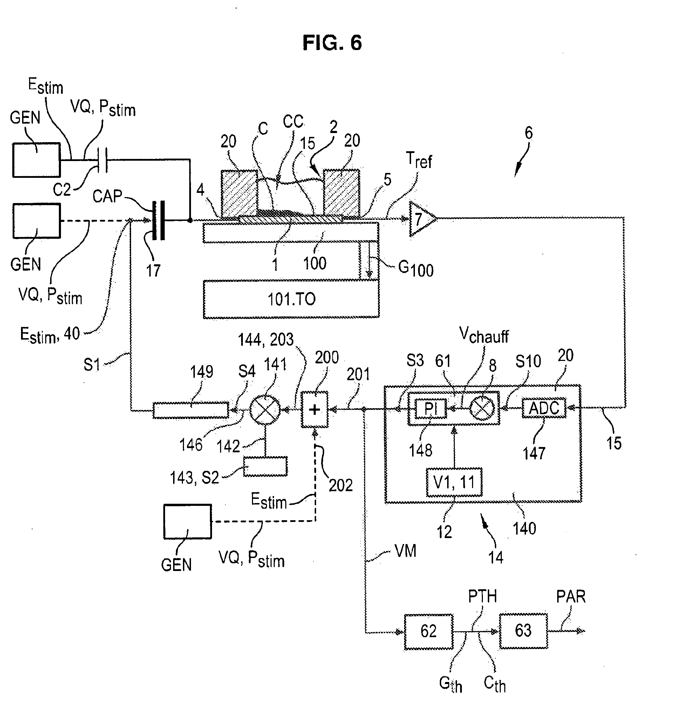

La figure 6 est une variante de la figure 5, où le signal de différence $V_{chauff}$ est numérique et est formé après conversion du signal analogique de température présent sur la sortie 9 en un signal numérique dans le convertisseur analogique-numérique 147, la sortie 9 étant reliée directement à l'entrée 15. Les éléments 8, 11 et 15 sont numériques pour former la différence numérique $V_{chauff}$ entre la sortie 9 numérisée à la sortie S10 du convertisseur 147 et le signal VI numérique constant fourni par un module numérique 12. Le signal de différence numérique $V_{chauff}$ est envoyé au module 148. La valeur numérique V1 constante est une valeur de consigne préenregistrée dans une mémoire 12 du microcontrôleur ou autres.

A la figure 6, l'élément 61 de mesure de la quantité d'énergie de chauffage est formé par l'élément 148 fournissant sur sa sortie le signal S3 qui est représentatif de la quantité d'énergie fournie par la boucle 6 à la résistance 1.

Le multiplieur 141 peut être soit relié directement à la sortie du module 148 pour recevoir le signal S3 sur l'entrée 144, soit être relié comme représenté à la sortie du module 148 par l'intermédiaire du module additionneur 200 tel que décrit ci-dessus en référence à la figure 5, pour avoir sur l'entrée 144 la somme du signal S3 et du signal $P_{stim}$, l'entrée 202 du module additionneur 200 étant reliée à l'entrée $E_{stim}$ de signal $P_{stim}$ de stimulation extérieure.

Grâce à l'invention, la détection des cellules est intégrée sur le support de culture. On évite ainsi le stress imposé aux cultures cellulaires, dû aux manipulations des cultures (sortie incubateur). On diminue les contraintes sur l'acquisition de données, en se dispensant de l'utilisation d'un microscope en environnement confiné. On n'a plus besoin d'une observation optique des cellules, ni de coloration ou de fluorescence. Un monitoring hautement parallèle en temps réel est rendu possible.

D'autre avantages sont également la simplicité du capteur 1, la facilité d'intégration, l'absence d'interaction électrique, le faible coût et le fait que le contrôle est non-invasif.

Liste de documents mentionnés :

[1] "Monitoring cell cycle by impedance spectroscopy: experimental and theoretical aspects" Eugen Gheorghiu, Koji Asami, Bioelectrochemistry and Bioenegetics 45 (1998) 139-143 ;

[2] "Analysis of the 3-omega method for thermal conductivity measurement" Hainan Wang, Mihir Sen, International Journal of Heat and Mass Transfer 52 (2009) 2102-2109 ;

[3] "Determination of thermal conductivity in liquids by monitoring transient phenomenon" Juraj Bozicevic, Alojz Caharija, Nena Bolf, Denis S. Vedrina, Procedings, XVII IMEKO World Congress, 22-27 juin 2003, Dubrovnik, Croatie ;

[4] "A micromachined AC thermal sensor for monitoring the liquid-gas interface in a microchannel", Sun Rock Choi, Jonggan Hong, Dongsik Kim, Sensors and Actuators A 150 (2009) 40-45 ;

[5] "Fibroplast Cells: A Sensing Bioelement for Glucose Detection by Impedance Spectroscopy", Chaker Tlili, Karine Reybier, Alain Géloën, Laurence Ponsonnet, Claude Martelet, Hafedh Ben Ouada, Michel Lagarde et Nicole Jaffrezzic-Renault, Anal. Chem. 2003, 75, 3340-3344.

**Revendications**

1. Dispositif de contrôle de l'évolution d'au moins une culture cellulaire, comportant une surface de réception de la culture cellulaire, la surface de réception étant en contact avec une impédance électrique agencée pour avoir une valeur temporelle d'impédance influencée par la présence de cellules dans la culture cellulaire, **caractérisé en ce que**
l'impédance comprend au moins une résistance électrique (1) de chauffage en contact avec la surface de réception pour le chauffage de la culture cellulaire (CC), la résistance étant reliée à un circuit électrique de fourniture d'énergie de chauffage à la résistance (1), la résistance (1) étant variable en fonction de la température et se trouvant dans une boucle (6) d'asservissement de chaleur formant le circuit électrique de chauffage pour maintenir à une température prescrite (Tref) de consigne la culture cellulaire (CC), la boucle (6) d'asservissement de chaleur comportant au moins un élément (61) de mesure d'au moins un premier paramètre (VM) représentatif de la quantité d'énergie de chauffage consommée par le circuit électrique, cet élément (61) de mesure servant également d'élément (61) de mesure de l'évolution de la culture cellulaire, l'élément (61) de mesure étant distinct de la résistance (1), le dispositif comportant en outre au moins un calculateur (62, 63) pour calculer à partir du premier paramètre (VM) représentatif de la quantité d'énergie de chauffage consommée par le circuit électrique au moins un deuxième paramètre (PAR) représentatif de l'évolution temporelle de la culture cellulaire (CC).

2. Dispositif suivant la revendication 1, **caractérisé en ce qu'**il comporte un générateur (GEN) d'un signal ($P_{stim}$) prédéterminé de stimulation thermique extérieure relié à un point (C2, 40) déterminé de la boucle (6) d'asservissement de chaleur, le calculateur (62, 63) étant prévu pour calculer à partir du premier paramètre (VM) représentatif de la quantité d'énergie de chauffage consommée par le circuit électrique au moins un troisième paramètre thermique (Pth), le troisième paramètre thermique (Pth) étant calculé par le calculateur (62, 63) à partir de la réponse de la résistance (1) au signal ($P_{stim}$) de stimulation thermique extérieure, ledit deuxième paramètre (PAR) représentatif de l'évolution temporelle de la culture cellulaire (CC) étant calculé par le calculateur (62, 63) à partir dudit troisième paramètre thermique (Pth).

3. Dispositif suivant la revendication 2, **caractérisé en ce que** le paramètre thermique (Pth) comprend la capacité thermique de la résistance (1) lorsqu'elle est en présence de la culture cellulaire (CC) et/ou la conductance thermique (Gth) de la résistance (1) lorsqu'elle est en présence de la culture cellulaire (CC).

4. Dispositif suivant l'une quelconque des revendications 2 et 3, **caractérisé en ce que** le signal ($P_{stim}$) de stimulation thermique extérieure est appliqué à l'une (4) des bornes de la résistance (1) par l'intermédiaire d'au moins une capacité électrique (17, C2).

5. Dispositif suivant l'une quelconque des revendications 2 et 3, **caractérisé en ce que** le signal ($P_{stim}$) de stimulation thermique extérieure est appliqué à un module additionneur (200) comportant une première entrée (201) sur laquelle se trouve le signal (VM) de mesure, une deuxième entrée (202) reliée au point déterminé de la boucle (6) d'asservissement de chaleur, auquel est appliqué le signal ($P_{stim}$) de stimulation extérieure, et une sortie (203) fournissant un signal d'asservissement de la résistance (1) égal à la somme des signaux présents sur la première entrée (201) et sur la deuxième entrée (202).

6. Dispositif suivant l'une quelconque des revendications 2 à 5, **caractérisé en ce que** le signal ($P_{stim}$) de stimulation thermique extérieure est sous la forme d'un signal électrique ayant une composante fréquentielle de moyenne nulle.

7. Dispositif suivant l'une quelconque des revendications 2 à 5, **caractérisé en ce que** le signal ($P_{stim}$) de stimulation thermique extérieure est sous la forme d'un signal électrique impulsionnel.

8. Dispositif suivant l'une quelconque des revendications 1 à 7, **caractérisé en ce que** la résistance électrique (1) de chauffage forme au moins en partie la surface de réception de la culture cellulaire (CC).

9. Dispositif suivant l'une quelconque des revendications 1 à 7, **caractérisé en ce que** la surface de réception de la culture cellulaire (CC) est prévue sur un matériau électriquement isolant interposé entre la résistance électrique (1) de chauffage et la culture cellulaire (CC).

10. Dispositif suivant la revendication 9, **caractérisé en ce que** le matériau électriquement isolant interposé entre la résistance électrique (1) de chauffage et la culture cellulaire (CC) est fixé de manière amovible par rapport à la résistance électrique (1) de chauffage.

**11.** Dispositif suivant l'une quelconque des revendications précédentes, **caractérisé en ce que** la résistance (1) est métallique avec une épaisseur comprise entre 5 et 20 nanomètres.

**12.** Dispositif suivant la revendication 11, **caractérisé en ce que** la résistance (1) comporte une première extrémité (110) reliée à un premier contact électrique (4) et une deuxième extrémité (120) reliée à un deuxième contact électrique (5), la résistance (1) ayant entre la première extrémité (110) et la deuxième extrémité (120) une valeur résistive (R) variable en fonction de la température, la valeur résistive (R) de la résistance (1) étant supérieure à la valeur résistive des contacts électriques (4, 5) formés chacun par au moins une couche métallique d'une épaisseur totale comprise entre 50 et 150 nanomètres.

**13.** Dispositif suivant l'une quelconque des revendications précédentes, **caractérisé en ce que** la résistance électrique (1) de chauffage forme au moins en partie le fond ou est disposée sous le fond d'au moins un puits (2) de culture contenant ladite au moins une culture cellulaire (CC).

**14.** Dispositif suivant la revendication 13, **caractérisé en ce qu'**une pluralité de puits (2) de culture destinés à contenir une pluralité de cultures cellulaires (CC) est disposée sur un support comportant une pluralité de résistances électriques (1) de chauffage des cultures cellulaires (CC), les résistances électriques (1) de chauffage étant agencées pour former au moins en partie le fond des puits (2) de culture ou étant disposées sous le fond des puits (2) de culture.

**15.** Dispositif suivant l'une quelconque des revendications précédentes, **caractérisé en ce que** la température prescrite (Tref) de consigne est constante et égale à une température déterminée de croissance de cellules vivantes (C) dans la culture cellulaire (CC).

**16.** Dispositif suivant l'une quelconque des revendications précédentes, **caractérisé en ce que** le deuxième paramètre (PAR) représentatif de l'évolution temporelle de la culture cellulaire (CC) est l'aire couverte par les cellules (C) de la culture cellulaire (CC) sur la surface de réception ou le pourcentage d'aire couverte par les cellules (C) de la culture cellulaire (CC) par rapport à l'aire de la surface de réception.

**17.** Procédé de contrôle de l'évolution d'au moins une culture cellulaire à l'aide du dispositif de contrôle suivant l'une quelconque des revendications précédentes, dans lequel une culture cellulaire est disposée sur une surface de réception de la culture cellulaire, la surface de réception étant en contact avec une impédance électrique agencée pour avoir une valeur temporelle d'impédance influencée par la présence de cellules dans la culture cellulaire, **caractérisé en ce que** l'impédance comprend une résistance électrique (1) de chauffage,
l'on chauffe par la résistance électrique (1) de chauffage en contact avec la surface de réception la culture cellulaire (CC), la résistance étant reliée à un circuit électrique de fourniture d'énergie de chauffage à la résistance (1),
la résistance (1) étant variable en fonction de la température et se trouvant dans une boucle (6) d'asservissement de chaleur formant le circuit électrique de chauffage pour maintenir à une température prescrite (Tref) de consigne la culture cellulaire (CC),
on mesure par un élément (61) de mesure de la boucle (6) d'asservissement de chaleur au moins un premier paramètre (VM) représentatif de la quantité d'énergie de chauffage consommée par le circuit électrique, cet élément (61) de mesure servant également d'élément (61) de mesure de l'évolution de la culture cellulaire, l'élément (61) de mesure étant distinct de la résistance (1),
on calcule par au moins un calculateur (62, 63) à partir du premier paramètre (VM) représentatif de la quantité d'énergie de chauffage consommée par le circuit électrique au moins un deuxième paramètre (PAR) représentatif de l'évolution temporelle de la culture cellulaire (CC).

**Patentansprüche**

**1.** Vorrichtung zur Überwachung der Entwicklung von zumindest einer Zellkultur, welche eine Fläche zur Aufnahme der Zellkultur aufweist, wobei die Aufnahmefläche in Kontakt mit einer elektrischen Impedanz steht, die angeordnet ist, um einen Zeitwert der Impedanz, der durch die Anwesenheit von Zellen in der Zellkultur beeinflusst wird, zu erlangen,
**dadurch gekennzeichnet, dass**
die Impedanz wenigstens einen elektrischen Heizwiderstand (1) in Kontakt mit der Aufnahmefläche zur Erwärmung der Zellkultur (CC) umfasst, wobei der Widerstand mit einem Stromkreis zur Versorgung des Widerstands (1) mit Wärmeenergie verbunden ist, wobei der Widerstand (1) in Abhängigkeit der Temperatur veränderlich ist und sich in einem Wärmeregelkreis (6) befindet, welcher den Heizstromkreis bildet, um die Zellkultur (CC) bei einer vorgeschriebenen Solltemperatur (Tref) zu halten, wobei der Wärmeregelkreis wenigstens ein Element (61) zur Messung wenigstens eines ersten Parameters (VM) umfasst, der repräsentativ für die Menge an Wärmeenergie, die durch den Stromkreis verbraucht wird, ist, wobei dieses Messelement (61) ebenso als Element (61) zur Messung der Entwicklung der Zellkultur dient, wobei das Messelement (61) getrennt von dem Widerstand (1) vorliegt, wobei die Vorrichtung außerdem wenigstens einen Rechner (62, 63) aufweist, um, ausgehend von dem ersten Parameter (VM), der repräsentativ für die Menge an Wärmeenergie, die durch den Stromkreis verbraucht wird, ist, wenigstens einen zweiten Parameter (PAR), der repräsentativ für die zeitliche Entwicklung der Zellkultur (CC) ist, zu berechnen.

2.  Vorrichtung gemäß Anspruch 1, **dadurch gekennzeichnet, dass** sie einen Generator (GEN) eines vorbestimmten Signals ($P_{stim}$) zur äußeren Wärmestimulation aufweist, der mit einem bestimmten Punkt (C2, 40) des Wärmeregelkreises (6) verbunden ist, wobei der Rechner geeignet ist, ausgehend von dem ersten Parameter (VM), der repräsentativ für die Menge an Wärmeenergie, die durch den Stromkreis verbraucht wird, ist, wenigstens einen dritten thermischen Parameter (Pth) zu berechnen, wobei der dritte Wärmeparameter (Pth) durch den Rechner (62, 63) ausgehend von der Antwort des Widerstands (1) auf das äußere Wärmestimulationssignal ($P_{stim}$) berechnet wird, der zweite Parameter (PAR), der repräsentativ für die zeitliche Entwicklung der Zellkultur (CC) ist, durch den Rechner (62, 63) ausgehend von dem dritten thermischen Parameter (Pth) berechnet wird.

3.  Vorrichtung gemäß Anspruch 2, **dadurch gekennzeichnet, dass** der thermische Parameter (Pth) die Wärmekapazität des Widerstands (1) bei Anwesenheit der Zellkultur (CC) und/oder die Wärmeleitfähigkeit (Gth) des Widerstands (1) bei Anwesenheit der Zellkultur (CC) umfasst.

4.  Vorrichtung gemäß irgendeinem der Ansprüche 2 und 3, **dadurch gekennzeichnet, dass** das Signal ($P_{stim}$) zur äußeren Wärmestimulation an einen (4) der Anschlusspunkte des Widerstands (1) über wenigstens eine elektrische Kapazität (17, C2) angelegt wird.

5.  Vorrichtung gemäß irgendeinem der Ansprüche 2 und 3, **dadurch gekennzeichnet, dass** das Signal ($P_{stim}$) zur äußeren Wärmestimulation an ein Addiermodul (200) angelegt wird, das einen ersten Eingang (201), an dem sich das Messsignal (VM) befindet, einen zweiten Eingang (202), der mit dem bestimmten Punkt des Wärmeregelkreises (6) verbunden ist, an den das äußere Stimulationssignal ($P_{stim}$) angelegt wird, und einen Ausgang (203), welcher ein Steuerungssignal des Widerstands (1) liefert, das der Summe der am ersten Eingang (201) und am zweiten Eingang (202) vorliegenden Signale entspricht, aufweist.

6.  Vorrichtung gemäß irgendeinem der Ansprüche 2 bis 5, **dadurch gekennzeichnet, dass** das Signal ($P_{stim}$) zur äußeren Wärmestimulation in Form eines elektrischen Signals mit einer Frequenzkomponente mit dem Mittelwert Null vorliegt.

7.  Vorrichtung gemäß irgendeinem der Ansprüche 2 bis 5, **dadurch gekennzeichnet, dass** das Signal ($P_{stim}$) zur äußeren Wärmestimulation in Form eines elektrischen Impulses vorliegt.

8.  Vorrichtung gemäß irgendeinem der Ansprüche 1 bis 7, **dadurch gekennzeichnet, dass** der elektrische Heizwiderstand (1) wenigstens einen Teil der Fläche zur Aufnahme der Zellkultur (CC) bildet.

9.  Vorrichtung gemäß irgendeinem der Ansprüche 1 bis 7, **dadurch gekennzeichnet, dass** die Fläche zur Aufnahme der Zellkultur (CC) auf einem elektrisch isolierenden Material, welches zwischen dem elektrischen Heizwiderstand (1) und der Zellkultur (CC) angeordnet ist, vorgesehen ist.

10. Vorrichtung gemäß Anspruch 9, **dadurch gekennzeichnet, dass** das elektrisch isolierende Material, das zwischen dem elektrischen Heizwiderstand (1) und der Zellkultur (CC) angeordnet ist, ablösbar in Bezug auf den elektrischen Heizwiderstand (1) fixiert ist.

11. Vorrichtung gemäß irgendeinem der vorhergehenden Ansprüche, **dadurch gekennzeichnet, dass** der Widerstand (1) ein Metall mit einer Stärke von 5 bis 20 Nanometern ist.

12. Vorrichtung gemäß Anspruch 11, **dadurch gekennzeichnet, dass** der Widerstand (1) ein erstes Ende (110), welches mit einem ersten elektrischen Kontakt (4) verbunden ist, und ein zweites Ende (120), welches mit einem zweiten elektrischen Kontakt (5) verbunden ist, aufweist, wobei der Widerstand (1) zwischen dem ersten Ende (110) und dem zweiten Ende (120) einen ohmschen Wert (R) aufweist, der in Abhängigkeit von der Temperatur veränderlich ist, wobei der ohmsche Wert (R) des Widerstands (1) höher ist als der ohmsche Wert der elektrischen Kontakte (4, 5), die jeweils durch wenigstens eine Metallschicht mit einer Gesamtdicke von 50 bis 150 Nanometern gebildet werden.

**13.** Vorrichtung gemäß irgendeinem der vorhergehenden Ansprüche, **dadurch gekennzeichnet, dass** der elektrische Heizwiderstand (1) wenigstens teilweise den Boden einer Kulturkavität (2) bildet oder unter dem Boden wenigstens einer Kulturkavität (2), welche die wenigstens eine Zellkultur (CC) enthält, angeordnet ist.

**14.** Vorrichtung gemäß Anspruch 13, **dadurch gekennzeichnet, dass** eine Vielzahl an Kulturkavitäten (2), die dazu bestimmt sind, eine Vielzahl an Zellkulturen (CC) zu enthalten, auf einem Träger angeordnet ist, welcher eine Vielzahl an elektrischen Heizwiderständen (1) der Zellkulturen (CC) aufweist, wobei die elektrischen Heizwiderstände (1) derart angeordnet sind, dass sie wenigstens teilweise den Boden der Kulturkavitäten (2) bilden, oder unter den Kulturkavitäten (2) angeordnet sind.

**15.** Vorrichtung gemäß irgendeinem der vorhergehenden Ansprüche, **dadurch gekennzeichnet, dass** die vorgeschriebene Solltemperatur (Tref) konstant ist und einer bestimmten Wachstumstemperatur der lebenden Zellen (C) in der Zellkultur (CC) entspricht.

**16.** Vorrichtung gemäß irgendeinem der vorhergehenden Ansprüche, **dadurch gekennzeichnet, dass** der zweite Parameter (PAR), der repräsentativ für die zeitliche Entwicklung der Zellkultur (CC) ist, der Bereich, der von den Zellen (C) der Zellkultur (CC) auf der Aufnahmefläche bedeckt wird, oder der prozentuale Anteil des Bereichs, der von den Zellen (C) der Zellkultur (CC) in Bezug auf den Bereich der Aufnahmefläche bedeckt wird, ist.

**17.** Verfahren zur Überwachung der Entwicklung wenigstens einer Zellkultur mit Hilfe einer Überwachungsvorrichtung gemäß wenigstens einem der vorhergehenden Ansprüche, in der eine Zellkultur auf einer Fläche zur Aufnahme der Zellkultur angeordnet ist, wobei die Aufnahmefläche in Kontakt mit einer elektrischen Impedanz steht, die angeordnet ist, um einen Zeitwert der Impedanz, der durch die Anwesenheit von Zellen in der Zellkultur beeinflusst wird, zu erlangen,
**dadurch gekennzeichnet, dass**
die Impedanz wenigstens einen elektrischen Heizwiderstand (1) umfasst,
man durch den elektrischen Heizwiderstand (1) in Kontakt mit der Aufnahmefläche die Zellkultur (CC) erwärmt, wobei der Widerstand mit einem Stromkreis zur Lieferung von Wärmeenergie an den Widerstand (1) verbunden ist, wobei der Widerstand (1) in Abhängigkeit von der Temperatur veränderlich ist und sich in einem Wärmeregelkreis (6) befindet, der den Heizstromkreis bildet, um die Zellkultur (CC) bei einer vorgeschriebenen Solltemperatur (Tref) zu halten,
man durch ein Messelement (61) des Wärmeregelkreises (6) wenigstens einen ersten Parameter (VM) misst, der repräsentativ für die Menge an Wärmeenergie, die durch den Stromkreis verbraucht wird, ist, wobei dieses Messelement (61) ebenso als Element (61) zur Messung der Entwicklung der Zellkultur dient, wobei das Messelement (61) getrennt von dem Widerstand (1) vorliegt,
man durch wenigstens einen Rechner (62, 63), ausgehend von dem ersten Parameter (VM), der repräsentativ für die Menge an Wärmeenergie, die durch den Stromkreis verbraucht wird, ist, wenigstens einen zweiten Parameter (PAR), der repräsentativ für die zeitliche Entwicklung der Zellkultur (CC) ist, berechnet.

**Claims**

**1.** A device for monitoring the development of at least one cell culture, comprising a receiving surface for receiving the cell culture, the receiving surface being in contact with electrical impedance arranged to have an impedance time value influenced by the presence of cells in the cell culture,
**characterised in that**
the impedance comprises at least one electrical heating resistor (1) in contact with the receiving surface for the heating of the cell culture (CC), the resistor being connected to an electrical circuit for supplying heating energy to the resistor (1), the resistor (1) being variable as a function of the temperature and located in a heat control loop (6) forming the electric heating circuit to maintain the cell culture (CC) at a prescribed setpoint temperature (Tref), the heat control loop (6) comprising at least one measuring element (61) for measuring at least one first parameter (VM) representative of the quantity of heat energy consumed by the electrical circuit, this measuring element (61) also serving as measuring element (61) for measuring the development of the cell culture, the measuring element (61) being distinct from the resistor (1), the device further comprising at least one calculator (62, 63) for calculating at least one second parameter (PAR) representative of the time development of the cell culture (CC) from the first parameter (VM) representative of the quantity of heat energy consumed by the electrical circuit.

2. The device as claimed in Claim 1, **characterised in that** it comprises a generator (GEN) of a predetermined signal ($P_{stim}$) of external thermal stimulation connected to a determined point (C2, 40) of the heat control loop (6), the calculator (62, 63) being provided for calculating at least one third thermal parameter (Pth) from the first parameter (VM) representative of the quantity of heat energy consumed by the electrical circuit, the third thermal parameter (Pth) being calculated by the calculator (62, 63) from the response of the resistor (1) to the signal ($P_{stim}$) of external thermal stimulation, said second parameter (PAR) representative of the time development of the cell culture (CC) being calculated by the calculator (62, 63) from said third thermal parameter (Pth).

3. The device as claimed in Claim 2, **characterised in that** the thermal parameter (Pth) comprises the thermal capacity of the resistor (1) when it is in the presence of the cell culture (CC) and/or the thermal conductance (Gth) of the resistor (1) when it is in the presence of the cell culture (CC).

4. The device as claimed in any one of Claims 2 and 3, **characterised in that** the signal ($P_{stim}$) of external thermal stimulation is applied to one (4) of the terminals of the resistor (1) by means of at least one electric capacitor (17, C2).

5. The device as claimed in any one of Claims 2 and 3, **characterised in that** the signal ($P_{stim}$) of external thermal stimulation is applied to an adding module (200) comprising a first input (201) on which the measuring signal (VM) is located, a second input (202) connected to the determined point of the heat control loop (6), to which the signal ($P_{stim}$) of external stimulation is applied, and an output (203) supplying a servo signal of the resistor (1) equal to the sum of the signals present on the first input (201) and on the second input (202).

6. The device as claimed in any one of Claims 2 to 5, **characterised in that** the signal ($P_{stim}$) of external thermal stimulation is in the form of an electric signal having a frequential component of zero average.

7. The device as claimed in any one of Claims 2 to 5, **characterised in that** the signal ($P_{stim}$) of external thermal stimulation is in the form of an electric impulse signal.

8. The device as claimed in any one of Claims 1 to 7, **characterised in that** the electrical heating resistor (1) forms at least partly the receiving surface of the cell culture (CC).

9. The device as claimed in any one of Claims 1 to 7, **characterised in that** the receiving surface of the cell culture (CC) is provided on electrically insulating material interposed between the electrical heating resistor (1) and the cell culture (CC).

10. The device as claimed in Claim 9, **characterised in that** the electrically insulating material interposed between the electrical heating resistor (1) and the cell culture (CC) is fixed removably relative to the electrical heating resistor (1).

11. The device as claimed in any one of the preceding claims, **characterised in that** the resistor (1) is metallic with a thickness of between 5 and 20 nanometres.

12. The device as claimed in Claim 11, **characterised in that** the resistor (1) comprises a first end (110) connected to a first electrical contact (4) and a second end (120) connected to a second electrical contact (5), the resistor (1) having between the first end (110) and the second end (120) a resistive value (R) variable as a function of the temperature, the resistive value (R) of the resistor (1) being greater than the resistive value of the electrical contacts (4, 5) formed each by at least one metallic layer of a total thickness of between 50 and 150 nanometres.

13. The device as claimed in any one of the preceding claims, **characterised in that** the electrical heating resistor (1) forms at least partly the bottom or is arranged under the bottom of at least one culture well (2) containing said at least one cell culture (CC).

14. The device as claimed in Claim 13, **characterised in that** a plurality of culture wells (2) intended to contain a plurality of cell cultures (CC) is arranged on a support comprising a plurality of electrical heating resistors (1) of cell cultures (CC), the electrical heating resistors (1) being arranged to form at least partly the bottom of the culture wells (2) or being arranged under the bottom of the culture wells (2).

15. The device as claimed in any one of the preceding claims, **characterised in that** the prescribed setpoint temperature (Tref) is constant and equal to a determined temperature for growth of living cells (C) in the cell culture (CC).

16. The device as claimed in any one of the preceding claims, **characterised in that** the second parameter (PAR) representative of the time development of the cell culture (CC) is the area covered by the cells (C) of the cell culture (CC) on the receiving surface or the percentage of area covered by the cells (C) of the cell culture (CC) relative to the area of the receiving surface.

17. A process for monitoring the development at least one cell culture by means of the device as claimed in any one of the preceding claims, in which a cell culture is arranged on a receiving surface for receiving of the cell culture, the receiving surface being in contact with electrical impedance arranged to have an impedance time value influenced by the presence of cells in the cell culture,
**characterised in that** the impedance comprises an electrical heating resistor (1),
the cell culture (CC) is heated by the electrical heating resistor (1) in contact with the receiving surface, the resistor being connected to an electrical circuit for supplying heating energy to the resistor (1),
the resistor (1) being variable as a function of the temperature and located in a heat control loop (6) forming the electric heating circuit to maintain the cell culture (CC) at a prescribed setpoint temperature (Tref),
at least one first parameter (VM) representative of the quantity of heat energy consumed by the electrical circuit is measured by a measuring element (61) of the heat control loop (6), this measuring element (61) also serving as measuring element (61) for measuring the development of the cell culture, the measuring element (61) being distinct from the resistor (1),
at least one second parameter (PAR) representative of the time development of the cell culture (CC) is calculated by at least one calculator (62, 63) from the first parameter (VM) representative of the quantity of heat energy consumed by the electrical circuit.

# FIG. 1

# FIG. 2

# FIG. 3

## FIG. 4

## FIG. 5

# FIG. 6

## FIG. 7

$P_{stim}$

$E_{stim}$

15

F(p)

G

V(T)

## FIG. 8

$P_{stim}$

$E_{stim}$

15

6

F(p)

G

V(T)

$P_{asser}$

H(p)

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009034066 A1

**Littérature non-brevet citée dans la description**

- **EUGEN GHEORGHIU ; KOJI ASAMI.** Monitoring cell cycle by impedance spectroscopy: experimental and theoretical aspects. *Bioelectrochemistry and Bioenegetics,* 1998, vol. 45, 139-143
- **HAINAN WANG ; MIHIR SEN.** Analysis of the 3-omega method for thermal conductivity measurement. *International Journal of Heat and Mass Transfer,* 2009, vol. 52, 2102-2109
- **JURAJ BOZICEVIC ; ALOJZ CAHARIJA ; NENA BOLF ; DENIS S. VEDRINA.** Determination of thermal conductivity in liquids by monitoring transient phenomenon. *Procedings, XVII IMEKO World Congress,* 22 Juin 2006
- **SUN ROCK CHOI ; JONGGAN HONG ; DONGSIK KIM.** A micromachined AC thermal sensor for monitoring the liquid-gas interface in a microchannel. *Sensors and Actuators A,* 2009, vol. 150, 40-45
- **CHAKER TLILI ; KARINE REYBIER ; ALAIN GÉLOËN ; LAURENCE PONSONNET ; CLAUDE MARTELET ; HAFEDH BEN OUADA ; MICHEL LAGARDE ; NICOLE JAFFREZZIC-RENAULT.** Fibroplast Cells: A Sensing Bioelement for Glucose Detection by Impedance Spectroscopy. *Anal. Chem.,* 2003, vol. 75, 3340-3344